# FASCICULE DE BREVET EUROPEEN

(11) **EP 3 082 567 B1**
(45) Date de publication et mention de la délivrance du brevet: **28.08.2019**
(21) Numéro de dépôt: 14827839.3
(22) Date de dépôt: 15.12.2014
(51) Int. Cl.: A61B 3/028, A61B 3/04, A61B 3/113, A61B 3/14, A61B 3/00, A61B 3/103, A61B 3/032

(54) **DISPOSITIF ET PROCÉDÉ DE MESURE DE LA RÉFRACTION SUBJECTIVE**
VORRICHTUNG UND VERFAHREN ZUR MESSUNG VON SUBJEKTIVER REFRAKTION
DEVICE AND METHOD FOR MEASURING SUBJECTIVE REFRACTION

(30) Priorité: 17.12.2013 FR 1362834
(43) Date de publication de la demande: 26.10.2016
(73) Titulaire: Essilor International, 94220 Charenton-le-Pont (FR)
(72) Inventeur: ROUSSEAU, Benjamin, 94227 Charenton le Pont (FR); HERNANDEZ, Martha, 94227 Charenton le Pont (FR); BARANTON, Konogan, 94227 Charenton le Pont (FR); OURIVES, Pedro, 94227 Charenton le Pont (FR); MARIN, Gildas, 94227 Charenton le Pont (FR)
(74) Mandataire: Laget, Jean-Loup
(86) Numéro de dépôt international: PCT/FR2014/053335
(87) Numéro de publication internationale: WO 2015/092244

(56) Documents cités:
- WO-A2-2013/123044
- DE-A1- 4 117 754
- FR-A1- 2 450 096
- US-A- 2 635 502
- US-A- 5 104 214
- US-A1- 2004 263 782

## Description

L'invention concerne un dispositif de mesure de la réfraction subjective en vision de près ou en vision intermédiaire. Le contexte de l'invention se situe dans la prise de mesure de paramètres à indiquer sur une prescription optique pour une personne nécessitant un équipement à verres correcteurs, de manière à corriger des défauts de la vision tels que la myopie, l'hypermétropie, l'astigmatisme et/ou la presbytie. L'invention concerne également un procédé de mesure de la réfraction subjective en vision de près et/ou en vision intermédiaire.

Généralement, et dans le cadre de cette description, on associe avec la vision de près des distances jusqu'à 70 cm, et avec la vision intermédiaire des distances d'environ 70 cm à quatre mètres. On peut, par exemple, associer la vision de près à la lecture d'un livre ou un Smartphone tenu dans les mains, et la vision intermédiaire à la vision sur un écran d'ordinateur, un téléviseur, un tableau de bord d'une voiture, ou aux activités ménagères.

Des dispositifs et méthodes pour mesurer la réfraction subjective d'une personne sont connus. On utilise notamment une tête de réfracteur pour déterminer le type (sphère et/ou cylindre) et la puissance (mesurée en dioptries) des verres correcteurs nécessaires. Cette tête de réfracteur fait partie d'un ensemble appelé unité de réfraction. La tête de réfracteur est un dispositif ayant à l'intérieur un support de verres (ou support de correction) consistant en deux trous et pouvant accueillir des verres correcteurs. La tête de réfracteur dispose de degrés de liberté de translation pour bien positionner les verres correcteurs devant les yeux de la personne. Pour mesurer la réfraction de la vision de loin, la personne regarde droit devant elle à travers les trous un tableau optométrique placé à l'infini (par exemple, sur un mur à environ 5 à 6 m). Ensuite, pour mesurer la vision de près, le patient regarde, à travers le réfracteur, un plan vertical de lecture, par exemple affichant un motif, placé à une distance fixe d'environ 40 cm. Pour procéder à ces mesures, la direction du regard de la personne ne change pas par rapport à sa tête. Le professionnel place des verres correcteurs de différents types et puissances dans le support de verres et effectue des réglages (par exemple, pour adapter l'écart interpupillaire) afin de déterminer le verre correcteur ou la combinaison de verres offrant la meilleure vision possible, en se basant sur le retour subjectif du patient. Typiquement, pour déterminer la prescription optique pour la vision de près, on ajoute une correction additionnelle (consistant en au moins un verre correcteur) à au moins un verre correcteur correspondant à la prescription pour la vision de loin.

Des dispositifs connus sont décrits, par exemple, dans US 5 104 214 A, US 2 635 502 A, DE 41 17 745 A1 et US 2004/0263782. Le document US 2004/0263782, en particulier, qui représente l'état de la technique le plus proche décrit un dispositif tel que divulgué dans la préambule de la revendication 1.

Cependant, la technique décrite ci-dessus ne tient pas compte de la convergence des lignes de regard de la personne en vision de près. En effet, quand la personne regarde un plan de lecture en vision de près, son regard est convergent, la convergence signifiant que les yeux sont tournés de façon à ce que leurs axes se croisent sur le plan de lecture. En vision intermédiaire, le regard est également convergent dans une moindre mesure. Dans les deux cas, le regard peut également être abaissé. En conséquence, pendant la mesure en vision de près et/ou intermédiaire avec la méthode et les dispositifs connus, la personne est susceptible de ne pas regarder de manière bien centrée à travers les verres correcteurs, ce qui peut engendrer des aberrations et ainsi fausser les valeurs de la prescription optique. Il est alors possible que les lunettes prescrites à la personne ne soient pas adaptées de manière optimale à la vision de la personne. En outre, la technique décrite ne mesure pas la modification de cylindre que peut entraîner l'abaissement du regard.

La présente invention a pour objectif de remédier aux inconvénients cités ci-dessus en proposant un dispositif de mesure de la réfraction subjective d'une personne en vision de près et/ou intermédiaire tenant compte de la convergence du regard de la personne, afin de lui permettre d'adopter une posture naturelle pendant la mesure et de minimiser ou éviter les aberrations optiques. L'invention propose également un procédé de mesure de la réfraction subjective d'une personne en vision de près et/ou intermédiaire.

Selon un premier aspect, la présente invention concerne un dispositif de mesure de la réfraction subjective d'une personne en vision de près et/ou intermédiaire, le dispositif comprenant, pour chaque oeil de la personne, un support de correction équipé d'au moins une lentille de correction, les supports de correction étant agencés de manière à ce que la personne regarde à travers les lentilles.

La principale caractéristique du dispositif selon l'invention est que la position de chaque support de correction est variable pour régler un angle entre les axes des lentilles.

Un tel dispositif permet de mesurer la réfraction subjective en vision de près et/ou intermédiaire pour une personne étant dans une position naturelle, les axes des yeux étant convergents, grâce à l'adaptabilité de l'angle entre les axes des lentilles et ainsi de l'angle entre les plans des supports de correction, à savoir les plans des lentilles. La prescription optique déterminée ou confirmée à l'aide du dispositif selon l'invention permet alors de concevoir un équipement à verres correcteurs parfaitement bien adapté à la vision de la personne, notamment en vision de près et intermédiaire. De plus, les réglages du support de correction permettent de parfaitement adapter le dispositif à la morphologie de la personne.

Avantageusement, le dispositif comprend en outre des moyens de détermination de la direction du regard, de manière à ajuster les positions relatives des supports de correction pour faire converger les directions du regard des deux yeux sous un angle de convergence, de manière à guider le regard de la personne sur un motif.

De préférence, l'angle entre les axes des lentilles est réglable entre 0° et 30°. Ceci permet de tenir compte de différents écarts interpupillaires et différentes distances entre les yeux et le motif. L'angle entre les plans des supports de correction, qui correspond à l'angle de convergence, peut donc varier, de préférence, entre 150° et 180°.

Préférentiellement, chaque support de correction est ajustable en inclinaison. Ainsi, les directions du regard peuvent être abaissées selon un angle pantoscopique.

Avantageusement, le dispositif selon l'invention comprend en outre un support de lecture agencé à une distance et un angle variables par rapport aux supports de correction, le support de lecture étant destiné à afficher le motif.

Selon un mode de réalisation avantageux, les moyens de détermination de la direction du regard comprennent, pour chaque oeil, un viseur destiné à être mis en place devant l'oeil au moyen du support de correction.

De manière préférentielle, le viseur comporte un élément d'entrée et un élément de sortie aptes à être alignés selon la direction du regard entre l'oeil et la cible.

Par exemple, le viseur peut comprendre un cylindre ayant une surface cylindrique opaque ou dépolie. Les faces d'entrée et de sortie du cylindre peuvent, par exemple, comprendre un trou de visée ou une mire.

Selon un autre mode de réalisation avantageux, les moyens de détermination de la direction du regard comprennent des moyens d'acquisition et de traitement d'images. De préférence, les moyens de détermination de la direction du regard comprennent également un moyen d'émission de lumière émettant dans la direction des yeux de la personne.

Préférentiellement, le dispositif selon l'invention comprend en outre des moyens de réglage pour réduire l'écart entre la position des supports de correction et une position de consigne des supports de correction, les positions étant relatives au motif, dans lequel la position de consigne correspond à une position des supports de correction dans laquelle la personne regarde de façon centrée et perpendiculaire à travers les lentilles.

Avantageusement, le dispositif selon la présente invention comprend un moyen de discrimination des deux yeux. Ainsi, il est possible de mesurer la réfraction subjective pour chaque oeil indépendamment.

Selon un second aspect, la présente invention concerne également un procédé de mesure de la réfraction subjective d'une personne en vision de près et/ou intermédiaire, au moyen d'un dispositif selon l'invention, le procédé comprenant une étape de mise en place, devant chaque oeil de la personne au moyen du support de correction, d'au moins une lentille de correction correspondant à la prescription de la vision de près et/ou intermédiaire.

Les principales caractéristiques du procédé selon l'invention sont les étapes suivantes :
- variation des positions des supports de correction pour régler un angle entre les axes des lentilles ; et
- détermination de l'acuité visuelle de la personne en vision de près et/ou intermédiaire.

De manière particulièrement avantageuse, le procédé selon l'invention comprend en outre les étapes suivantes :
- détermination de la direction du regard des deux yeux ; et
- ajustement des positions relatives des supports de correction pour faire converger les directions du regard sous un angle de convergence, de manière à guider le regard de la personne sur le motif.

Selon un premier mode de réalisation avantageux, lequel l'étape de détermination de la direction du regard comprend une étape de mise en place, devant chaque l'oeil au moyen du support de correction, du viseur.

Selon un deuxième mode de réalisation avantageux, l'étape de détermination de la direction du regard comprend une étape de détermination du centre de rotation de l'oeil.

Selon un troisième mode de réalisation avantageux, l'étape de détermination de la direction du regard comprend une étape de détermination des positions des pupilles.

Avantageusement, le procédé comprend en outre une étape d'inclinaison d'un plan contenant les supports de correction pour abaisser les directions du regard selon un angle pantoscopique.

Avantageusement, l'étape de la mesure de l'acuité visuelle en vision de près et/ou intermédiaire comprend les étapes suivantes :
- mise en place de moyens de discrimination des deux yeux ; et
- mesure de l'acuité visuelle en vision de près et/ou intermédiaire pour chaque oeil indépendamment.

De façon préférentielle, le procédé selon l'invention comprend en outre une étape de réglage itératif, au moyen de moyens de réglage, de la position des supports de correction pour réduire l'écart entre la position des supports de correction et une position de consigne des supports de correction, les positions étant relatives au motif, dans lequel la position de consigne correspond à une position des supports de correction dans laquelle la personne regarde de façon centrée et perpendiculaire à travers les lentilles.

L'invention et les avantages qu'elle procure seront mieux compris au vu de la description suivante de modes de réalisation de l'invention, donnés à titre d'exemple et en référence aux figures annexées, dans lesquelles :
- la figure 1 montre une partie d'un dispositif de mesure de la réfraction subjective d'une personne en vision de près et/ou intermédiaire selon un mode de réalisation de l'invention ;
- la figure 2A montre, à titre d'exemple, un support de tête et des supports de correction équipés de cylindres de visée selon un mode de réalisation de l'invention ;
- la figure 2B illustre schématiquement une vue de côté des supports de correction ayant un angle d'inclinaison par rapport à la verticale, selon un mode de réalisation ;
- la figure 3A représente schématiquement un cylindre de visée selon un mode de réalisation de l'invention ;
- la figure 3B illustre l'angle de convergence entre des axes des cylindres de visée ;
- la figure 4A montre une vue d'un support de lecture du dispositif selon un mode de réalisation ; et
- la figure 4B montre, à titre d'exemple, une vue de face du support de lecture.

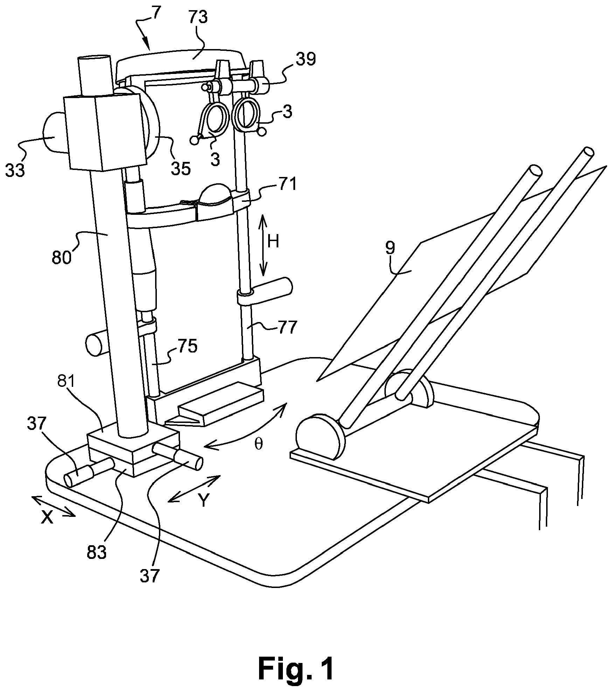

Selon un premier aspect, la présente invention propose un dispositif de mesure de la réfraction subjective d'une personne, ou d'un patient, en vision de près et/ou intermédiaire. La figure 1 montre une partie d'un dispositif de mesure de la réfraction subjective d'une personne en vision de près et/ou intermédiaire selon un mode de réalisation de l'invention.

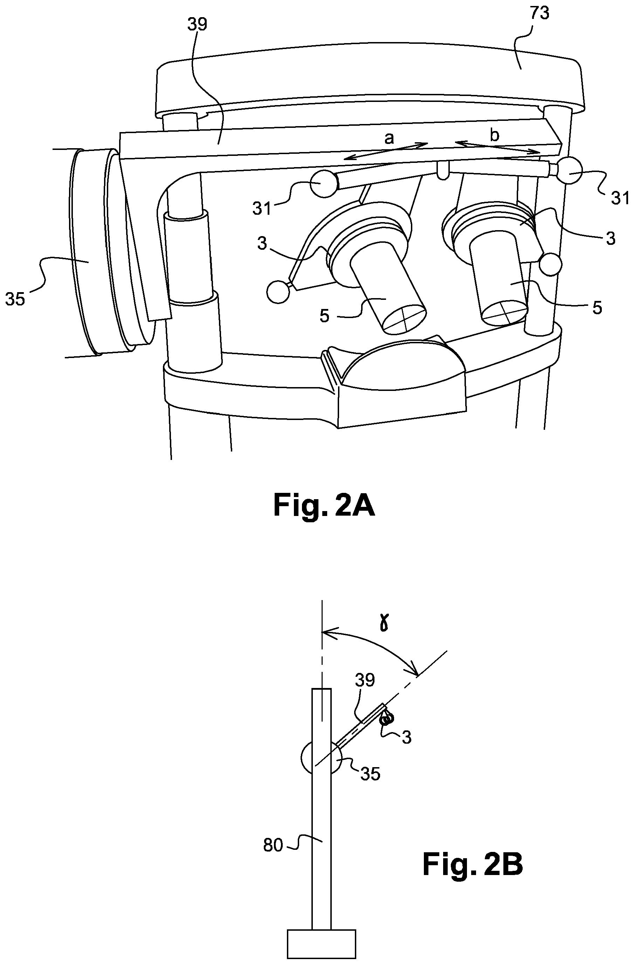

Le dispositif comprend, pour chaque oeil de la personne, un support 3 de correction destiné à recevoir au moins une lentille de correction (non représentée). Les supports 3 de correction sont agencés de manière à ce que la personne regarde à travers la lentille de correction pour chaque oeil. Les supports 3 de correction peuvent être, par exemple, du type d'une monture d'essai. Une vue plus détaillée des supports 3 de correction est présentée sur la figure 2A.

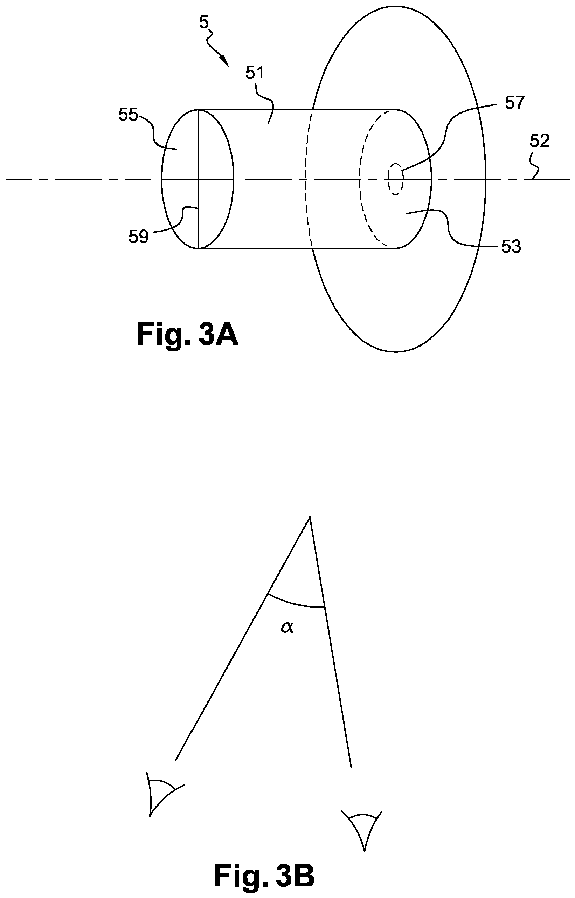

En se référant à la figure 2A, le dispositif selon le mode de réalisation présenté comprend en outre, pour chaque oeil de la personne, un viseur 5 défini par un axe et destiné à être mis en place devant l'oeil au moyen du support de correction 3. Les positions relatives des supports 3 de correction sont ajustables pour faire converger les axes des viseurs, de manière à guider le regard de la personne sur un motif (non représenté) permettant la mesure de l'acuité visuelle en vision de près et/ou intermédiaire. Par « acuité visuelle », on comprend la performance visuelle au sens large, par exemple : acuité visuelle à proprement parler, sensibilité au contraste, sensibilité au flou, appréciation subjective de la qualité d'image. Par « positions relatives », on comprend la position relative de l'un des supports 3 de correction par rapport à l'autre. Par exemple, sur la figure 2A, les deux supports 3 de correction sont basculés l'un par rapport à l'autre. Les positions relatives permettent de définir l'angle de convergence α entre les axes des viseurs 5 (voir également figure 3B). Ainsi, pour un angle de convergence α de zéro, les supports 3 de correction se trouvent dans le même plan. L'angle de convergence α varie en fonction de l'écart pupillaire (IPD) de la personne dont la réfraction subjective est mesurée et une distance de visée (D) entre les supports 3 de correction et le motif, et peut être exprimé comme suit : α = arctan(IPD/D). À titre d'exemple, pour une distance de visée de 40 cm et une distance pupillaire de 64 mm, l'angle de convergence α est d'environ 9,1° ; pour une distance de visée de 5 m et une distance pupillaire de 64 mm, l'angle de convergence α est d'environ 0,7°. De préférence, l'angle α de convergence et donc l'angle entre les supports 3 de correction est réglable entre 0° et 30°.

Le motif peut être constitué par des caractères tels que des lettres, des optotypes connus tels qu'un anneau de Landolt ou par toute autre figure permettant de mesurer l'acuité visuelle. Le motif peut également être un test duochrome, par exemple une lettre noire sur un fond rouge ou vert. Il peut également être un test de sensibilité au contraste, test de phories, de disparités, de vision binoculaire.

Les viseurs 5 peuvent être constitués, par exemple, par des cylindres 5, comme montré sur la figure 2A. La figure 3A illustre schématiquement et plus en détails un cylindre viseur 5 selon un mode de réalisation préféré. Le cylindre 5 est creux. Il présente un axe 52 principal, une surface cylindrique latérale 51 dépolie ou opaque, une face 53 circulaire d'entrée qui se trouve près de l'oeil et une face circulaire 55 de sortie. La face 53 d'entrée est constituée par une rondelle ayant un diamètre plus grand que le cylindre 5, qui permet au cylindre 5 d'être tenu dans le support 3 de correction (voir figure 2A). La rondelle peut être dépolie ou opaque. Selon le mode de réalisation illustré sur la figure 3A, la face 53 d'entrée comprend un trou 57 de visée afin de guider le regard dans le cylindre 5. Le trou 57 de visée a un effet sténopéïque qui augmente la profondeur de champ. La face 55 de sortie comprend une mire 59 en forme de croix pour guider le regard sur le motif. Alternativement, la face 53 d'entrée peut également comprendre, à la place du trou de visée, une mire. A titre d'exemple, la longueur du cylindre 5 est de l'ordre de 3 cm, son diamètre de l'ordre de 1 cm, et le diamètre du trou 57 de l'ordre de 2 mm.

Le viseur 5 permet de limiter le regard à l'axe du viseur 5. En pratique, les positions des supports 3 de correction sont réglées à l'aide des viseurs 5 installés dans les supports 3 de correction. Ainsi, les axes du regard de la personne sont bien déterminés et seront confondus avec les axes optiques des lentilles de corrections. Ensuite, la ou les lentilles de corrections insérées dans les supports 3 de correction. Ainsi, les positions des supports 3 de correction sont réglées de façon à ce que la lentille ou les lentilles de correction soient placées dans les supports 3 de correction avec leurs axes coïncidant avec celui de chaque oeil. Le patient regarde donc à travers le(s) verre(s) de correction de manière droite et centrée. La prescription optique déterminée ou confirmée à l'aide du dispositif selon l'invention permet alors de concevoir un équipement à verres correcteurs parfaitement bien adapté à la vision de la personne, notamment en vision de près et intermédiaire.

De manière particulièrement avantageuse, les positions des supports 3 de correction peuvent donc être ajustées pour faire coïncider, pour chaque oeil, l'axe de l'oeil et l'axe 52 du viseur 5. Le ou les verres de correction destinés à être placés dans les supports 3 de correction sont donc également parfaitement centrés par rapport aux yeux du patient.

En référence au mode de réalisation représenté à la figure 1, le dispositif de mesure de la réfraction subjective comprend également un support 7 de tête afin que le patient puisse poser sa tête derrière les supports 3 de correction. Typiquement, le support 7 de tête consiste en une mentonnière 71 et une structure 73 offrant appui au front, les deux structures 71, 73 étant reliées par un premier montant fixe 75 et un deuxième montant fixe 77. Les montants 75, 77 sont fixés sur une table. Il est également possible de fixer les montants 75, 77 sur une platine (non représentée) de manière à pouvoir pivoter la mentonnière 71 et la structure 73 d'appui du front autour de l'axe vertical selon un angle θ. Il est également possible d'incliner la structure de support 7 de tête autour d'un axe horizontal (non représenté) de manière à incliner la tête en avant, l'angle d'inclinaison pouvant être, par exemple, de 20°. Les structures 71, 73 de support du menton et du front sont coulissants sur les premier et deuxième montants 75, 77 afin de pouvoir adapter leur hauteur à la morphologie du patient. Comme illustré sur les figures 1 et 2A, les supports 3 de correction peuvent être installés à l'aide d'un bras mobile 39 de manière pivotante sur un troisième montant 80 indépendant du support de tête 7. Les supports 3 de correction peuvent pivoter conjointement autour d'un axe horizontal, pour être inclinés avec un angle d'inclinaison γ par rapport à la verticale (voir figure 2B), indépendamment de la position du support 7 de tête. Ceci permet aux supports 3 de correction d'être bien en face des yeux de la personne afin que les axes de ses yeux (ou du regard) coïncident avec les axes des viseurs 5, quelles que soient les orientations relatives de la tête et des yeux de la personne. L'angle γ est aussi appelé angle pantoscopique des supports 3 de correction.

En se référant à la figure 1, le dispositif de mesure de la réfraction subjective comprend en outre un support 9 de lecture agencé à une distance et un angle variables par rapport aux supports 3 de correction. Le support 9 de lecture est adapté pour afficher le motif.

De manière avantageuse, le dispositif de mesure de la réfraction subjective comprend des moyens de réglage pour réduire l'écart entre la position des supports 3 de correction et une position de consigne des supports 3 de correction, les positions étant relatives au motif. Par « position », on doit comprendre à la fois la distance et l'angle d'inclinaison (angle pantoscopique) γ des supports 3 de correction par rapport au motif et le support 9 de lecture qui l'affiche. La position de consigne correspond à une position (en termes de distance et d'angle) par rapport au motif dans laquelle la personne regarde de façon perpendiculaire à travers les verres correcteurs placés dans les supports 3 de correction pour voir le motif de façon nette.

Plus précisément et en se référant aux figures 1 et 2A, les supports 3 de correction sont pourvus de moyens 31 de réglage pour adapter leur distance à la distance pupillaire de la personne (flèches a et b), de moyens 33 de réglage de la hauteur (flèche h) et de moyens de réglage (non représentés) de l'angle de convergence α entre les axes des viseurs 5. Les supports 3 de correction comprennent en outre de moyens 35 de réglage pour ajuster l'inclinaison γ des supports 3 de correction par rapport à l'horizontale, et des moyens 37 de réglage de translation dans les deux directions horizontales (flèches x et y). Les moyens 37 de réglage de translation peuvent être constitués par deux platines en translation 81, 83 aptes à translater le troisième montant 80 portant les supports 3 de correction. Le support 9 de lecture est également pourvu de moyens de réglage, comme détaillé par la suite.

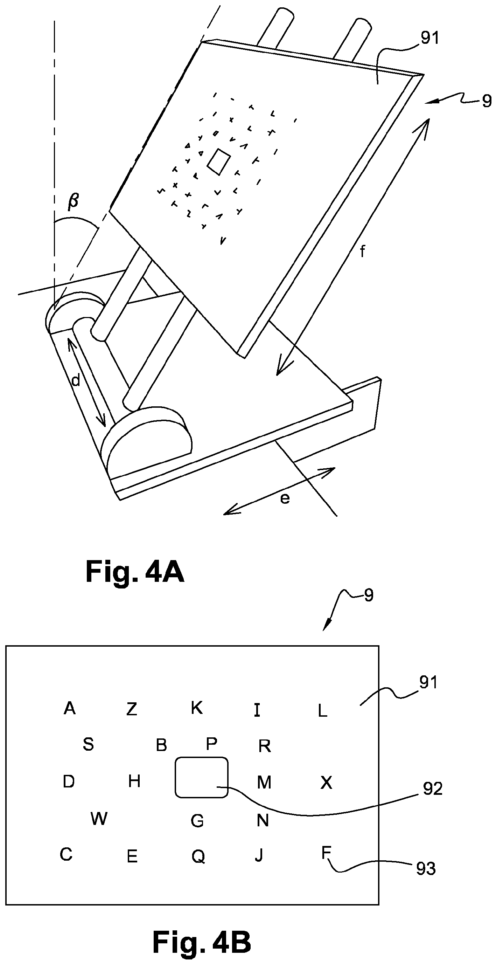

Les figures 4A et 4B montrent des vues différentes du support 9 de lecture du dispositif selon un mode de réalisation préféré de l'invention. Le support 9 de lecture comprend un plateau 91 sur lequel est agencé un moyen 92 d'affichage apte à afficher le motif. Le moyen 92 d'affichage peut être un moyen d'affichage électronique, par exemple, un écran à cristaux liquides ou tout autre type d'écran plat. La taille du moyen d'affichage est de préférence au minimum d'environ 2mm × 2mm à 20mm x 20mm pour la partie centrale en vision de près. Un affichage de 20cm x 20cm permettra d'inclure une partie périphérique et sera plus approprié pour des distances en vision intermédiaire plus grande. L'avantage d'un moyen d'affichage électronique est que le type, la taille et d'autres caractéristiques du motif affiché peuvent être variés ou adaptés en temps réel pendant l'utilisation du dispositif de mesure de la réfraction subjective selon l'invention. À titre d'exemple, pour une distance de visée de 40 cm, le motif affiché, tel qu'une lettre, peut être < 1mm, par exemple, 0,8 mm.

Autour du moyen 92 d'affichage sont agencés des signes graphiques ou caractères 93 (tels que des lettres ou des chiffres). Les signes graphiques 93 peuvent être directement imprimés sur le plateau 91, ou ils peuvent figurer sur une feuille de papier ayant un trou au niveau du moyen 92 d'affichage et venant se poser ou se fixer sur le plateau 91.

Les caractères 93 sont significatifs de l'endroit sur le plateau 91 où ils se trouvent et permettent ainsi de connaitre la direction du regard de la personne regardant le support 9 de lecture. Pour que la personne regarde le moyen 92 d'affichage, la position du support 9 de lecture peut être ajustée en translation et en rotation. Comme indiqué par des flèches (d, e, f) sur la figure 4A, le support 9 de lecture peut notamment être translaté dans les directions perpendiculaires (d, e) à la direction du regard.

Selon un mode de réalisation préféré, le dispositif comporte des moyens de discrimination des deux yeux. Ces moyens peuvent, par exemple, être constitués par un couple polariseur-analyseur entre le support de correction et le motif. Le moyen 92 d'affichage peut comprendre, par exemple, un affichage polarisé, tel qu'un écran à cristaux liquides. Ainsi, il est possible d'occulter la vue pour l'un des yeux du patient en tournant un polariseur, placé par exemple dans un des supports 3 de correction, de sorte que son axe principal soit perpendiculaire à la direction de polarisation des ondes lumineuses émises par l'écran. Ceci permet de procéder à la mesure de la réfraction subjective en vision de près ou intermédiaire pour chaque oeil indépendamment, sans couvrir mécaniquement l'autre oeil. Ainsi, les deux yeux regardent la cible, mais le motif ne peut être déchiffré que par un oeil à la fois, ce qui permet de ne mesurer l'acuité que d'un oeil à la fois tout en restant dans les conditions d'un regard binoculaire abaissé normal.

Alternativement, les moyens de discrimination entre l'oeil droit et l'oeil gauche peuvent être des moyens habituellement employés pour la vision stéréoscopique, comme par exemple des anaglyphes en combinaison avec des lunettes aux verres rouge et vert, des électroshutters ou des écrans auto-stéréoscopiques. Peuvent également être considérés les filtres directifs tels que les « filtres de confidentialité » (proposés, par exemple, par la société 3M Company).

Selon d'autres modes (non représentés) de réalisation avantageux du dispositif de la présente invention, les moyens de détermination de la direction du regard peuvent comprendre des moyens d'acquisition et de traitement d'images. Les moyens d'acquisition d'images peuvent être constitués, par exemple, par une caméra 92 placée sur le support 9 de lecture, à la place du motif décrit précédemment (voir figure 4B). Il est ainsi possible de déterminer la direction du regard, soit en déterminant le centre de rotation de l'oeil à l'aide de marqueurs d'échelle sur les supports de correction ou sur la mentonnière, soit en déterminant les positions des pupilles en observant les reflets de lumière sur les cornées. Dans le second cas, les moyens de détermination de la direction du regard comprennent également un moyen d'émission de lumière émettant dans la direction des yeux de la personne, par exemple une diode électroluminescente (LED).

Selon un deuxième aspect, la présente invention concerne un procédé de mesure de la réfraction subjective d'une personne en vision de près et/ou intermédiaire. Avantageusement, le procédé est mis en oeuvre au moyen d'un dispositif de mesure selon les modes de réalisation décrits ci-dessus. Le procédé comprend les étapes suivantes.

Devant chaque oeil de la personne, au moins une lentille de correction correspondant à la prescription de la vision de loin est mise en place au moyen d'un support de correction. Les supports 3 de correction peuvent être ceux décrits en relation avec les figures 1 et 2A. Il est ainsi possible de vérifier l'acuité visuelle en vision de loin. Pour la mesure de la réfraction subjective en vision de loin, le patient regarde un motif affiché sur un plan vertical ou un tableau optométrique à environ 5 à 6 m des supports 3 de correction, typiquement droit devant lui. Les supports 3 de correction ne sont pas inclinés par rapport à la verticale. L'angle de convergence α entre les deux supports de correction est ajusté à zéro, les deux supports 3 de correction ne sont donc pas basculés l'un par rapport à l'autre. En effet, comme vu ci-dessus, en vision de loin, la distance de visée étant d'environ 5 m à 6 m, l'angle de convergence entre les axes des yeux est proche de zéro.

Selon le procédé de l'invention, devant chaque oeil de la personne, au moins une lentille de correction correspondant à la prescription de la vision de près et/ou intermédiaire est ensuite mise en place au moyen du support de correction. Selon ce mode de réalisation, pour la vision de près, le support de correction porte, à la fin de la mesure, au moins les éléments suivants : la correction en vision de loin (à savoir, la correction sphérique et/ou cylindrique), la lentille de correction de sphère dite « d'addition » pour la vision de près, la lentille de correction de cylindre dit « d'addition » pour la vision de près, et un viseur.

Afin de déterminer la direction du regard de la personne et guider le regard sur le motif, le viseur est mis en place devant chaque oeil de la personne au moyen du support 3 de correction. Le viseur est de préférence un cylindre 5 comme décrit précédemment en relation avec la figure 3A. Les positions relatives des supports 3 de correction sont ainsi ajustées pour faire converger les axes des viseurs. Les positions des supports 3 de correction sont ajustées pour faire coïncider, pour chaque oeil, l'axe de l'oeil et l'axe du viseur 5. Ainsi, lorsque le regard de la personne est abaissé en vision de près, par exemple, les yeux de la personne restent parfaitement centrés par rapport au(x) verre(s) de correction.

L'acuité visuelle de la personne est ensuite mesurée en vision de près et/ou en vision intermédiaire. On peut retirer le viseur pour effectuer cette mesure.

Pour la mesure de la réfraction subjective en vision de près, le patient regarde le motif sur un support 9 de lecture placé de préférence à environ 40 cm des supports 3 de correction. Le support 9 de lecture peut comporter un moyen 92 d'affichage comme détaillé en référence aux figures 4A et 4B. En se référant à la figure 4A, la distance entre le support 9 de lecture et les supports 3 de correction peut être ajustée en translatant le support 9 de lecture (selon la direction indiquée par la flèche e). Typiquement, la personne abaisse son regard. Ceci correspond à la posture naturelle que prend une personne en vision de près, par exemple, pour la lecture. Afin que la personne regarde à travers les verres de correction, destinés à être mis en place dans les supports 3 de correction, de manière perpendiculairement centré, les supports 3 de correction doivent être inclinés de la même façon que les yeux à l'aide des viseurs 5, comme décrit ci-dessus. L'angle d'inclinaison γ des supports 3 de correction est d'environ 0 à 45° par rapport à la verticale, et de préférence environ 36°. Avantageusement, le support 9 de lecture est également incliné avec un angle β par rapport à la verticale d'environ 0 à 45°, et de préférence environ 36°. Dans un mode de réalisation préféré, le plan du support 9 de lecture et le plan contenant les supports 3 de correction sont substantiellement parallèles pour garantir une bonne visibilité du motif. Par ailleurs, on peut aussi incliner le support de tête (7) pour une posture plus naturelle.

Pour la mesure de la réfraction subjective en vision intermédiaire, le patient regarde le motif sur le support 9 de lecture placé de préférence à environ 70 cm à 100 cm des supports 3 de correction. De manière similaire à la vision de près, la personne peut abaisser son regard, sans bouger la tête. Ceci correspond à la posture naturelle que prend une personne, par exemple, pour effectuer des tâches ménagères ou regarder un écran d'ordinateur. Les supports 3 de correction doivent être inclinés de la même façon que les yeux à l'aide des viseurs 5, comme décrit ci-dessus. L'angle d'inclinaison γ pour la vision intermédiaire peut être d'une vingtaine de degrés par rapport à la verticale. Le support 9 de lecture est également incliné avec un angle β par rapport à la verticale.

Il est donc nécessaire que la distance entre les supports 3 de correction et le support 9 de lecture puisse être ajustée indépendamment des inclinaisons des supports 3 de correction et du support 9 de lecture.

La prise en compte de l'abaissement du regard de la personne par les inclinaisons et la hauteur du support 9 de lecture et des supports 3 de correction permet également de correctement évaluer l'éventuelle variation de la réfraction en sphère ou en astigmatisme de l'oeil. En effet, comme l'oeil tourne légèrement en s'abaissant, l'axe de la correction cylindrique n'est pas la même pour un regard droit (au loin) et un regard abaissé (sur un livre, par exemple). Le dispositif et le procédé selon des modes de réalisation décrits permettent de déterminer correctement la correction cylindrique nécessaire pour concevoir, par exemple, des verres correcteurs progressifs parfaitement adaptés à la fois à la vision de loin, la vision intermédiaire et la vision de prés.

Selon un mode de réalisation préféré du procédé selon l'invention, l'étape de la mesure de l'acuité visuelle en vision de près et/ou en vision intermédiaire comprend la discrimination des deux yeux. Selon un exemple, le motif est affiché sur un écran à cristaux liquides, et l'étape de la mesure de l'acuité visuelle comprend les étapes suivantes. Un polariseur est placé devant chaque oeil de la personne au moyen du support de correction. Le premier polariseur est réglé pour que le motif soit visible pour le premier oeil, et le second polariseur est réglé pour que le motif soit invisible pour le second oeil. Le motif est visible lorsque l'axe principal du polariseur coïncide avec la direction de polarisation des ondes lumineuses émises par l'écran. Le motif est invisible lorsque l'axe principal du polariseur est perpendiculaire à la direction de polarisation des ondes. L'acuité visuelle en vision de près et/ou intermédiaire (le mode étant déterminé par la distance du motif par rapport aux yeux) est mesurée ensuite pour le premier oeil. Ce procédé est répété pour le second oeil.

Selon des modes de réalisation avantageux du procédé de l'invention, au lieu d'utiliser des viseurs comme décrit précédemment, l'étape de détermination de la direction du regard peut être réalisée en déterminant soit le centre de rotation de l'oeil (CRO), soit les positions des pupilles de la personne.

Pour la mesure du CRO, le patient est positionné de manière définie (par exemple, avec une distance de visée de 40 cm et le regard abaissé de 36° par rapport à l'horizontale). Le patient regarde le centre de l'objectif de la caméra des photos sont prises avec différentes positions de la mentonnière 71 et l'appui-front 73 (par exemple, avec θ = 0°, 20°, -20°, voir figure 1). Les photos prises peuvent ensuite être analysées pour calculer les positions des CRO par rapport, par exemple, à la mentonnière 71 et aux supports 3 de correction, ces derniers étant avantageusement équipés de marqueurs d'échelles. Les supports 3 de correction peuvent ensuite être positionnés perpendiculairement et de façon centrée sur les droites passant par les CRO et le centre de l'objectif.

Pour la mesure des CRO, les photos peuvent aussi être prises par plusieurs caméras ou par une seule caméra mobile, le patient regardant successivement les caméras et la mentonnière restant fixe.

Pour la mesure des positions des pupilles, on observe les positions de reflets de lumière (émise, par exemple, par une LED située sur le support 9 de lecture et émettant dans le spectre visible ou infrarouge) par rapport aux supports 3 de correction, ces derniers étant équipés de mires marquant les centres des supports 3 de correction. Le patient est bien positionné quand les reflets sont bien centrés par rapport aux mires.

Préférentiellement, le procédé selon l'invention comprend en outre le réglage itératif, au moyen de moyens de réglage, de la position des supports 3 de correction pour réduire l'écart entre la position des supports 3 de correction et une position de consigne des supports 3 de correction, les positions étant relatives au motif. Comme décrit précédemment, les supports 3 de correction peuvent être réglés en hauteur, en translation sur le plan horizontal et l'un par rapport à l'autre pour adapter leur séparation à l'écart pupillaire du patient, et encore de manière rotative ou pivotante. Ces réglages peuvent être manuels ou automatisés.

En conséquence, et de manière très avantageuse, la détermination de la direction du regard du patient peut ainsi être effectuée de manière complètement automatisée. Par exemple, les réglages des différentes parties du support 7 de tête, détaillés ci-dessus, ainsi que ceux des supports 3 de correction peuvent être déterminés et commandés par un ordinateur pourvu d'une interface dédiée et opérés à l'aide de moyens de réglage motorisés. Les supports 3 de correction peuvent alors être correctement alignés et centrés par rapport aux axes de regard de la personne de façon sûre, rapide et efficace.

## Revendications

1. Dispositif de mesure de la réfraction subjective d'une personne en vision de près et/ou intermédiaire, comprenant :
- pour chaque oeil de la personne, un support (3) de correction équipé d'au moins une lentille de correction, les supports (3) de correction étant agencés de manière à ce que la personne regarde à travers les lentilles ; la position de chaque support (3) de correction étant variable pour régler un angle (α) entre les axes des lentilles,.
- des moyens (5) de détermination de la direction du regard, de manière à ajuster les positions relatives des supports (3) de correction pour faire converger les directions du regard des deux yeux sous un angle (α) de convergence, de manière à guider le regard de la personne sur un motif.
**Caractérisé en ce que** les moyens (5) comprennent des viseurs consistant en des cylindres creux comprenant une surface cylindrique latérale (51) dépolie ou opaque, une face (53) circulaire d'entrée qui se trouve près de l'oeil et une face circulaire (55) de sortie, et **en ce que** la face (53) d'entrée est constituée par une rondelle ayant un diamètre plus grand que le cylindre (5), permettant au cylindre (5) d'être tenu dans le support (3) de correction.

2. Dispositif selon la revendication 1, dans lequel l'angle (α) entre les axes des lentilles est réglable entre 0° et 30°.

3. Dispositif selon la revendication 1, dans lequel chaque support (3) de correction est ajustable en inclinaison.

4. Dispositif selon l'une des revendications 1 à 3, comprenant en outre un support (9) de lecture, agencé à une distance et un angle variable par rapport aux supports (3) de correction, le support (9) de lecture étant destiné à afficher le motif.

5. Dispositif selon l'une des revendications 1 à 4, dans lequel les moyens (5) de détermination de la direction du regard comprennent des moyens d'acquisition et de traitement d'images.

6. Dispositif selon la revendication 5, dans lequel les moyens (5) de détermination de la direction du regard comprennent en outre un moyen d'émission de lumière émettant dans la direction des yeux de la personne.

7. Dispositif selon l'une des revendications précédentes, comprenant en outre des moyens de réglage pour réduire l'écart entre la position des supports (3) de correction et une position de consigne des supports (3) de correction, les positions étant relatives au motif, dans lequel la position de consigne correspond à une position des supports (3) de correction dans laquelle la personne regarde de façon centrée et perpendiculaire à travers les lentilles.

8. Dispositif selon l'une des revendications précédentes, comprenant en outre des moyens de discrimination des deux yeux.

9. Procédé de mesure de la réfraction subjective d'une personne en vision de près et/ou intermédiaire, au moyen d'un dispositif selon l'une des revendications 1 à 8, le procédé comprenant une étape de :
mise en place, devant chaque oeil de la personne au moyen du support (3) de correction, d'au moins une lentille de correction correspondant à la prescription de la vision de près et/ou intermédiaire ; **caractérisé en ce que** le procédé comprend en outre les étapes suivantes :
- une étape de détermination de la direction du regard de la personne au moyen de l'insertion d'un viseur (5) sur chaque support (3) de correction, chaque viseur (5) se présentant sous la forme d'un cylindre creux comprenant une surface cylindrique latérale (51) dépolie ou opaque, une face (53) circulaire d'entrée qui se trouve près de l'oeil et une face circulaire (55) de sortie, la face (53) d'entrée étant constituée par une rondelle ayant un diamètre plus grand que le cylindre (5), permettant au cylindre (5) d'être tenu dans le support (3) de correction,
- une étape de variation des positions des supports (3) de correction pour régler un angle (α) entre les axes des lentilles, les positions des supports (3) de correction étant ajustées pour faire coïncider, pour chaque oeil, l'axe de l'oeil et l'axe du viseur (5),
- une étape de détermination de l'acuité visuelle de la personne en vision de près et/ou intermédiaire.

10. Procédé selon la revendication 9, comprenant en outre les étapes suivantes :
- une étape d'ajustement des positions relatives des supports (3) de correction pour faire converger les directions du regard sous un angle (α) de convergence, de manière à guider le regard de la personne sur le motif.

11. Procédé selon la revendication 9, dans lequel l'étape de détermination de la direction du regard comprend une étape de détermination du centre de rotation de l'oeil.

12. Procédé selon la revendication 9, dans lequel l'étape de détermination de la direction du regard comprend une étape de détermination des positions des pupilles.

13. Procédé selon l'une des revendications 9 à 12, comprenant en outre une étape d'inclinaison d'un plan contenant les supports (3) de correction pour abaisser les directions du regard selon un angle pantoscopique (γ).

14. Procédé selon l'une des revendications 9 à 13, dans lequel l'étape de la mesure de l'acuité visuelle en vision de près et/ou intermédiaire comprend les étapes suivantes :
- mise en place de moyens de discrimination des deux yeux ; et
- mesure de l'acuité visuelle en vision de près et/ou intermédiaire pour chaque oeil indépendamment.

15. Procédé selon l'une des revendications 9 à 14, comprenant en outre une étape de réglage itératif, au moyen de moyens de réglage, de la position des supports (3) de correction pour réduire l'écart entre la position des supports (3) de correction et une position de consigne des supports (3) de correction, les positions étant relatives au motif, dans lequel la position de consigne correspond à une position des supports (3) de correction dans laquelle la personne regarde de façon centrée et perpendiculaire à travers les lentilles.

## Patentansprüche

1. Vorrichtung zur Messung der subjektiven Refraktion einer Person in Nah- und/oder Zwischensicht, die enthält:
- für jedes Auge der Person einen Korrekturträger (3), der mit mindestens einer Korrekturlinse x ausgestattet ist, wobei die Korrekturträger (3) so eingerichtet sind, dass die Person durch die Linsen blickt; wobei die Position jedes Korrekturträgers (3) variabel ist, um einen Winkel (α) zwischen den Achsen der Linsen einzustellen,
- Einrichtungen (5) zur Bestimmung der Blickrichtung, um die relativen Positionen der Korrekturträger (3) anzupassen, um die Blickrichtungen der zwei Augen unter einem Konvergenzwinkel (α) konvergieren zu lassen, um den Blick der Person auf ein Muster zu lenken,
**dadurch gekennzeichnet, dass** die Einrichtungen (5) Sichtgeräte enthalten, die aus Hohlzylindern bestehen, die eine matte oder opake zylindrische Seitenfläche (51), eine kreisförmige Eingangsseite (53), die sich nahe dem Auge befindet, und eine kreisförmige Ausgangsseite (55) enthalten, und dass die Eingangsseite (53) aus einer Scheibe besteht, die einen größeren Durchmesser hat als der Zylinder (5), wodurch der Zylinder (5) im Korrekturträger (3) gehalten werden kann.

2. Vorrichtung nach Anspruch 1, wobei der Winkel (α) zwischen den Achsen der Linsen zwischen 0° und 30° einstellbar ist.

3. Vorrichtung nach Anspruch 1, wobei die Neigung jedes Korrekturträgers (3) anpassbar ist.

4. Vorrichtung nach einem der Ansprüche 1 bis 3, die außerdem einen Leseträger (9) enthält, der in variablem Abstand und Winkel bezüglich der Korrekturträger (3) angeordnet ist, wobei der Leseträger (9) dazu bestimmt ist, das Muster anzuzeigen.

5. Vorrichtung nach einem der Ansprüche 1 bis 4, wobei die Einrichtungen (5) zur Bestimmung der Blickrichtung Einrichtungen zur Erfassung und Verarbeitung von Bildern enthalten.

6. Vorrichtung nach Anspruch 5, wobei die Einrichtungen (5) zur Bestimmung der Blickrichtung außerdem eine Lichtemissionseinrichtung enthalten, die in Richtung der Augen der Person emittiert.

7. Vorrichtung nach einem der vorhergehenden Ansprüche, die außerdem Einstelleinrichtungen enthält, um die Abweichung zwischen der Position der Korrekturträger (3) und einer Sollposition der Korrekturträger (3) zu reduzieren, wobei die Positionen sich auf das Muster beziehen, wobei die Sollposition einer Position der Korrekturträger (3) entspricht, in der die Person mittig und lotrecht durch die Linsen blickt.

8. Vorrichtung nach einem der vorhergehenden Ansprüche, die außerdem Einrichtungen zur Unterscheidung der zwei Augen enthält.

9. Verfahren zur Messung der subjektiven Refraktion einer Person in Nah- und/oder Zwischensicht mittels einer Vorrichtung nach einem der Ansprüche 1 bis 8, wobei das Verfahren enthält:
- einen Schritt des Anordnens, vor jedem Auge der Person mittels des Korrekturträgers (3), mindestens einer Korrekturlinse, die der Verordnung der Nah- und/oder Zwischensicht entspricht;
**dadurch gekennzeichnet, dass** das Verfahren außerdem die folgenden Schritte enthält:
- einen Schritt der Bestimmung der Blickrichtung der Person mittels der Einfügung eines Sichtgeräts (5) auf jeden Korrekturträger (3), wobei jedes Sichtgerät (5) in Form eines Hohlzylinders vorliegt, der eine matte oder opake zylindrische Seitenfläche (51), eine kreisförmige Eingangsseite (53), die sich nahe dem Auge befindet, und eine kreisförmige Ausgangsseite (55) enthält, wobei die Eingangsseite (53) aus einer Scheibe besteht, die einen größeren Durchmesser hat als der Zylinder (5), wodurch der Zylinder (5) im Korrekturträger (3) gehalten werden kann,
- einen Schritt der Änderung der Positionen der Korrekturträger (3), um einen Winkel (α) zwischen den Achsen der Linsen einzustellen, wobei die Positionen der Korrekturträger (3) angepasst werden, um für jedes Auge die Achse des Auges und die Achse des Sichtgeräts (5) in Übereinstimmung zu bringen,
- einen Schritt der Bestimmung der Sehschärfe der Person in Nah- und/oder Zwischensicht.

10. Verfahren nach Anspruch 9, das außerdem die folgenden Schritte enthält:
- einen Schritt der Anpassung der relativen Positionen der Korrekturträger (3), um die Blickrichtungen unter einem Konvergenzwinkel (α) konvergieren zu lassen, um den Blick der Person auf das Muster zu lenken.

11. Verfahren nach Anspruch 9, wobei der Schritt der Bestimmung der Blickrichtung einen Schritt der Bestimmung des Drehpunkts des Auges enthält.

12. Verfahren nach Anspruch 9, wobei der Schritt der Bestimmung der Blickrichtung einen Schritt der Bestimmung der Positionen der Pupillen enthält.

13. Verlfahren nach einem der Ansprüche 9 bis 12, das außerdem einen Schritt der Neigung einer die Korrekturträger (3) enthaltenden Ebene enthält, um die Blickrichtungen gemäß einem pantoskopischen Winkel (γ) zu senken.

14. Verfahren nach einem der Ansprüche 9 bis 13, wobei der Schritt der Messung der Sehschärfe in Nah- und/oder Zwischensicht die folgenden Schritte enthält:
- Einsetzen von Unterscheidungseinrichtungen der zwei Augen; und
- Messen der Sehschärfe in Nah- und/oder Zwischensicht unabhängig für jedes Auge.

15. Verfahren nach einem der Ansprüche 9 bis 14, das außerdem einen Schritt der iterativen Einstellung mittels Einstelleinrichtungen der Position der Korrekturträger (3) enthält, um die Abweichung zwischen der Position der Korrekturträger (3) und einer Sollposition der Korrekturträger (3) zu reduzieren, wobei die Positionen sich auf das Muster beziehen, wobei die Sollposition einer Position der Korrekturträger (3) entspricht, in der die Person mittig und lotrecht durch die Linsen blickt.

## Claims

1. Device for measuring the near- and/or intermediate-vision subjective refraction of a person, comprising:
- for each eye of the person, a corrective holder (3) equipped with at least one corrective lens, the corrective holders (3) being arranged so that the person looks through the lenses; it being possible for the position of each corrective holder (3) to be varied to adjust an angle (α) between the axes of the lenses,
- means (5) for determining the direction of the gaze, so as to adjust the relative positions of the corrective holders (3) in order to make the directions of the gaze of the two eyes converge with an angle of convergence (α), so as to guide the gaze of the person onto a pattern, **characterized in that** the means (5) comprise sights consisting of hollow cylinders comprising an opaque or frosted lateral cylindrical surface (51), a circular entrance face (53) that is placed near the eye and a circular exit face (55), and **in that** the entrance face (53) consists of a disc having a larger diameter than the cylinder (5), which allows the cylinder (5) to be held in the corrective holder (3).

2. Device according to Claim 1, wherein the angle (α) between the axes of the lenses is adjustable between 0° and 30°.

3. Device according to Claim 1, wherein the inclination of each corrective holder (3) is regulatable.

4. Device according to one of Claims 1 to 3, furthermore comprising a reading support (9) arranged at a distance and angle that may be varied relative to the corrective holders (3), the reading support (9) being intended to display the pattern.

5. Device according to one of Claims 1 to 4, wherein the means (5) for determining the direction of the gaze comprise means for acquiring and processing images.

6. Device according to Claim 5, wherein the means (5) for determining the direction of the gaze furthermore comprise a light-emitting means emitting in the direction of the eyes of the person.

7. Device according to one of the preceding claims, furthermore comprising adjusting means for decreasing the deviation between the position of the corrective holders (3) and a setpoint position of the corrective holders (3), the positions being relative to the pattern, wherein the setpoint position corresponds to a position of the corrective holders (3) in which the person looks perpendicularly through the center of the lenses.

8. Device according to one of the preceding claims, furthermore comprising means for discriminating the two eyes.

9. Method for measuring the near- and/or intermediate-vision subjective refraction of a person, by means of a device according to one of Claims 1 to 8, the method comprising a step of:
placing, in front of each eye of the person, by means of the corrective holder (3), at least one corrective lens corresponding to his near- and/or intermediate-vision prescription; **characterized in that** the method furthermore comprises the following steps:
- a step of determining the direction of the gaze of the person by means of insertion of a sight (5) into each corrective holder (3), each sight (5) taking the form of a hollow cylinder comprising an opaque or frosted lateral cylindrical surface (51), a circular entrance face (53) that is placed near the eye and a circular exit face (55), the entrance face (53) consisting of a disc having a larger diameter than the cylinder (5), which allows the cylinder (5) to be held in the corrective holder (3),
- a step of varying the positions of the corrective holders (3) in order to adjust an angle (α) between the axes of the lenses, the positions of the corrective holders (3) being adjusted in order to make, for each eye, the axis of the eye and the axis of the sight (5) coincide,
- a step of determining the near- and/or intermediate-vision visual acuity of the person.

10. Method according to Claim 9, furthermore comprising the following steps:
- a step of adjusting the relative positions of the corrective holders (3) in order to make the directions of the gaze converge with an angle of convergence (α), so as to guide the gaze of the person onto the pattern.

11. Method according to Claim 9, wherein the step of determining the direction of the gaze comprises a step of determining eye rotation center.

12. Method according to Claim 9, wherein the step of determining the direction of the gaze comprises a step of determining positions of the pupils.

13. Method according to one of Claims 9 to 12, furthermore comprising a step of inclining a plane containing the corrective holders (3) in order to lower the directions of the gaze by a pantoscopic angle (γ).

14. Method according to one of Claims 9 to 13, wherein the step of measuring near- and/or intermediate-vision visual acuity comprises the following steps:
- placing means for discriminating the two eyes; and
- measuring the near- and/or intermediate-vision visual acuity for each eye independently.

15. The method according to one of Claims 9 to 14, furthermore comprising a step of iteratively adjusting, by means of adjusting means, the position of the corrective holders (3) in order to decrease the deviation between the position of the corrective holders (3) and a setpoint position of the corrective holders (3), the positions being relative to the pattern, wherein the setpoint position corresponds to a position of the corrective holders (3) in which the person looks perpendicularly through the center of the lenses.
